Europäisches Patentamt

European Patent Office

Office européen des brevets

(19)

(11) Publication number: **0 227 250 B1**

(12) **EUROPEAN PATENT SPECIFICATION**

(45) Date of publication of patent specification: **13.03.91**  (51) Int. Cl.⁵: **C07C 29/136, C07C 31/125**

(21) Application number: **86308231.9**

(22) Date of filing: **22.10.86**

(54) **Process for producing diisopropylcarbinol.**

(30) Priority: **21.11.85 JP 262234/85**

(43) Date of publication of application:
**01.07.87 Bulletin 87/27**

(45) Publication of the grant of the patent:
**13.03.91 Bulletin 91/11**

(84) Designated Contracting States:
**CH DE GB LI**

(56) References cited:
**BE-A- 869 056**
**US-A- 3 055 840**

(73) Proprietor: **Chisso Corporation**
**6-32, Nakanoshima 3-chome Kita-ku**
**Osaka-shi Osaka-fu(JP)**

(72) Inventor: **Tani, Hirotaka**
**17, Tatsumidaihigashi 2-chome**
**Ichiharashi Chibaken(JP)**
Inventor: **Saito, Kikutaro**
**157-9, Ichihara**
**Ichiharashi Chibaken(JP)**

(74) Representative: **Ruffles, Graham Keith et al**
**MARKS & CLERK 57-60 Lincoln's Inn Fields**
**London WC2A 3LS(GB)**

EP 0 227 250 B1

**Description**

BACKGROUND OF THE INVENTION

I. Field of the Invention

This invention relates to production of diisopropylcarbinol, and more particularly it relates to a process for producing diisopropylcarbinol by hydrogenating diisopropyl ketone with a ruthenium catalyst.

2. Description of the Related Art

Diisopropylcarbinol is a commercially useful substance as solvent, extraction agent, raw materials for polymerization initiator, perfume, medicine, pesticide, etc.

Diisopropylcarbinol has so far been obtained by subjecting diisopropyl ketone to hydrogenation reaction under gas-liquid contact in the presence of Raney nickel, but due to the so-called steric hindrance wherein carbonyl group to be hydrogenated is covered by two isopropyl groups, reaction conditions of high temperatures and high pressures are required. Further, in order to raise the conversion, a long reaction time has been required; hence the above hydrogenation process has been uneconomical. Still further, it is difficult to separate and remove unreacted diisopropylcarbinol; hence in order to obtain a high purity product, a fractionating column having 40 plates or more is required. Ruthenium-containing catalysts have been prepared for the reduction of some aldehydes and ketones - see US-A-3055840.

SUMMARY OF THE INVENTION

In order to overcome such drawbacks, the present inventors have made extensive research, and achieved the present invention.

The present invention resides in:

a process for producing diisopropylcarbinol of a high purity suitable as an industrial raw material with a high yield, by subjecting diisopropyl ketone to hydrogenation reaction by the use of a catalyst containing ruthenium as a catalytically active substance, under conditions of lower temperatures, lower pressures and shorter times than those of conventional process.

DETAILED DESCRIPTION OF PREFERRED EMBODIMENTS

As the catalyst containing ruthenium as a catalytically active substance, a commercially available product obtained by having 0.I to I0% by weight of ruthenium supported on a carrier such as activated carbon, diatomaceous earth or alumina, having a large surface area, followed by activation may be used. The shape may be either in pellet form for use in fixed bed or in powdery form for use in fluidized bed. In the case of powdery form, the catalyst containing ruthenium as a catalytically active substance may be either a dried product or a product containing water in a quantity to the same extent as that of the catalyst for its easy handling.

The reaction conditions where diisopropyl ketone is hydrogenated using the above catalyst may be established as follows:

Reaction may be advanced at a reaction temperature of 20 to 200°C, preferably 70 to I50°C and under a reaction pressure of the atmospheric pressure to $9.8 \times 10^5$ Pa (I00 Kg/cm$^2$) gauge, preferably $9.8 \times 10^5$ to $4.9 \times 10^6$ Pa (I0 to 50 Kg/cm$^2$) gauge. Diisopropyl ketone may be contacted with hydrogen gas either in liquid phase or gas phase. In the case of contact in liquid phase, methanol, ethanol, propanol, butanol, hexanol or octanol or saturated hydrocarbons may be used as diluent, but it is rather preferred not to use it, taking into consideration, the difficulty of the succeeding distillation and purification. In the hydrogenation reaction, the catalyst may be continuously used in fixed bed, or the catalyst may be non-continuously used in a state where powdery catalyst is suspended in diisopropyl ketone, in fluidized bed.

In the case of the non-continuous hydrogenation, usually this reaction is preferred to be carried out in a quantity of the catalyst containing ruthenium as its catalytically active substance of 0.I to I0 parts by weight, preferably 0.5 to 5 parts by weight based on I00 parts by weight of diisopropyl ketone, for a reaction time of

2

0.l to 9 hours, preferably 0.l to 7 hours.

In the case of the continuous hydrogenation, preferably this reaction is carried out at the abovementioned temperatures and pressures in a fixed bed reactor according to a conventional technique such as storage process or spray process. Diisopropyl ketone is preferably continuously fed to the reactor in a quantity of 0.l to 5 parts by volume/hr based on l00 parts by volume of the catalyst, by means of an injecting pump.

The fluid to be reacted may be once passed through the catalyst layer and immediately withdrawn to obtain a reaction fluid, or while the reaction fluid is circulated through the catalyst layer, the fluid to be reacted may be fed to the circulating fluid or directly to the catalyst layer, followed by withdrawing a portion of the reaction fluid. Further, the reaction fluid may be successively passed through a plurality of circulating apparatus containing the catalyst, the raw material being fed to the first circulating apparatus and the reaction fluid being withdrawn from the final circulating apparatus to obtain reaction fluid. The reaction fluid is directly fed to a fractionating column to be continuously operated, or collected in a storage vessel and then batchwise fractionated.

The present invention will be described in more detail by way of Examples.


Example l
‾‾‾‾‾‾‾

Diisopropyl ketone as raw material (purity: 99.l% according to gas chromatography (herein abbreviated to g.c.) (200 ml l58.8 g) was fed into a 300 ml autoclave and a catalyst of 5% Ru-carbon powder hydrous product (a product obtained by having 5% by weight of ruthenium supported on active carbon, followed by activating the resulting material and then having an equal quantity of water contained; a product made by Nippon Engelhard, LTD.) was fed in a quantity of 4% by weight (6.4 g) (the substantial catalyst quantity: 2% by weight, 3.2 g). After purging the autoclave with nitrogen and then with hydrogen, the pressure was raised up to $2.94 \times 10^6$ Pa (30 Kg/cm$^2$) gauge with hydrogen, followed by initiating temperature elevation with stirring at 500 rpm by means of a stirrer of electromagnetic induction type. Hydrogen absorption began notably at 40°C, and 53 minutes after the initiation of temperature elevation, the reaction temperature reached l30°C. While the reaction temperature was kept at l30°C, hydrogen absorption ceased 66 minutes after the initiation of temperature elevation. Further, the reaction was kept at $2.94 \times 10^6$ Pa (30 Kg/cm$^2$) gauge and 130°C for 22 minutes to complete the reaction. After cooling and pressure dropping, the autoclave was opened and the catalyst was filtered off under pressure, to obtain a reaction fluid (l55 g). According to g.c. analysis, the content of diisopropylcarbinol was 97.9% and the raw material diisopropyl ketone was completely extinct.

The catalyst had a still remaining activity, and even when it was repeatedly used (9 times) in the same manner except for the conditions described in Table l, almost no change was observed as shown in Table l (30 Kg/cm$^2$ = $2.94 \times 10^6$ Pa or 29.4 Bar)

## Table 1

| Experi-ment No. | Reaction tempera-ture °C | Reaction pressure Kg/cm$^2$G | Reaction time since start of temp. elevation till stop | Gas chromato-graphy GC% | |
|---|---|---|---|---|---|
| | | | | Diiso-propyl ketone | Diiso-propyl-carbinol |
| 1 | 130 | 30 | 1° 28′ | — | 97. 9 |
| 2 | 70 | 30 | 3° 15′ | — | 97. 5 |
| 3 | 80 | 30 | 2° 23′ | — | 97. 6 |
| 4 | 90 | 30 | 2° 26′ | — | 97. 7 |
| 5 | 100 | 30 | 1° 54′ | — | 97. 6 |
| 6 | 110 | 30 | 2° 09′ | — | 97. 7 |
| 7 | 120 | 30 | 2° 40′ | — | 97. 8 |
| 8 | 130 | 30 | 2° 32′ | 0. 07 | 97. 8 |
| 9 | 140 | 30 | 2° 49′ | 0. 16 | 97. 6 |
| 10 | 150 | 30 | 2° 55′ | 0. 18 | 97. 6 |

Example 2

Raw material diisopropyl ketone (g.c. purity: 99.1%) (200 mℓ, 158.8 g) was fed into a 300 mℓ autoclave, and a catalyst of 2% Ru-carbon powder dried product (made by Nippon Engelhard, LTD.) was fed in a quantity of 0.5% by weight (0.8 g) based on the raw material. After purging the autoclave with nitrogen and then with hydrogen, the pressure was raised up to 9.8x10$^5$ Pa (l0 Kg/cm$^2$) gauge with hydrogen, followed by initiating temperature elevation with stirring at 500 rpm by means of a stirrer of electromagnetic induction type. Thirty one minutes after the initiation of temperature elevation, the reaction temperature reached l00°C. While the reaction temperature was kept at l00°C, hydrogen absorption ceased 9 hours after the initiation of temperature elevation; thus it was regarded that the reaction was complete at that time. After cooling and pressure dropping, the autoclave was opened and the catalyst was filtered off under pressure to obtain a reaction fluid (l56 g). According to g.c. analysis, the content of diisopropylcarbinol was 97.0%, and that of unreacted diisopropyl ketone was 0.2%.

Example 3

Raw material diisopropyl ketone (g.c. purity: 99.I%) (200 mℓ, I58.8 g) was fed into a 300 mℓ autoclave, and a catalyst of 5% Ru-alumina powder dried product (made by Nippon Engelhard, LTD.) was fed in a quantity of 4% by weight (6.4 g) based on the raw material. After purging the autoclave with nitrogen and then with hydrogen, the pressure was raised up to $4.9 \times 10^5$ Pa (5 Kg/cm$^2$) gauge with hydrogen, and the mixture was agitated at 500 rpm by means of a stirrer of electromagnetic induction type. As a result, hydrogen absorption began. The reaction temperature was kept at 70°C and the pressure was kept at $4.9 \times 10^5$ Pa (5 Kg/cm$^2$) gauge. After 7 hours, hydrogen absorption ceased; thus it was regarded that the reaction was complete at that time. After cooling and pressure dropping, the autoclave was opened and the catalyst was filtered off to obtain a reaction fluid of I56 g. According to g.c. analysis, the content of diisopropylcarbinol was 97.6% and the raw material diisopropyl ketone was completely extinct.

Example 4

Into a I,200 mℓ capacity reactor (30 mm in diameter) was filled a catalyst of 0.5% Ru-diatomaceous earth molded product (cylindrical pellets of 5 mm in diameter and 5 mm high made by Heraeus Company) (I ℓ). After purging the autoclave with nitrogen and then with hydrogen, the pressure was raised up to $2.94 \times 10^6$ Pa (30 Kg/cm$^2$) gauge with hydrogen and the temperature was also raised up to I30°C. Raw material diisopropyl ketone (g.c. purity: 99.I%) was fed in a quantity of 200 mℓ/hr at the upper part of the reactor. Hydrogen was fed from below the catalyst layer and the pressure was kept at $2.94 \times 10^6$ Pa (30 Kg/cm$^2$) gauge. The resulting product after completion of the reaction was contained in a storage tank. The reaction fluid was sampled from the tank. According to g.c. analysis, the content of diisopropylcarbinol was 97.7% and the raw material diisopropyl ketone was completely extinct.

Comparative example I

Raw material diisopropyl ketone (g.c. purity: 99.I%) (200 mℓ, I58.8 g) was fed into a 300 mℓ autoclave, and a catalyst obtained by adding a small quantity of copper and chrome oxide as a cocatalyst to nickel supported by diatomaceous earth as a carrier, that is, a powdery product (N-II3B made by Nikki Kagaku Company) was fed in a quantity of 2% by weight (3.2 g) based on the raw material into the autoclave. Operation was carried out in the same manner as in Example I and temperature elevation was initiated. Thirty eight minutes after the initiation of temperature elevation, the reaction temperature reached I30°C, and reaction was carried out at this temperature. Seven hours after the initiation of temperature elevation, hydrogen absorption still continued slowly, but the reaction was stopped. After cooling and pressure dropping, the autoclave was opened and the catalyst was filtered off under pressure. Thereafter the weight of the reaction fluid was I55.5 g, and according to g.c. analysis, the content of diisopropylcarbinol was 40.7% and unreacted diisopropyl ketone remained in as large a quantity as 57.0%.

Comparative example 2

Operation was carried out in the same manner as in Example I except that a stabilized Raney nickel (DR●200, tradename of product made by Nikko Rika Company) was used as catalyst in a quantity of 2% by weight (3.2 g) based on the raw material, and temperature elevation was initiated. Thirty eight minutes after the initiation of temperature elevation, the reaction temperature reached I30°C. Thereafter, no hydrogen absorption occurred in this state for 30 minutes; thus, the temperature was further elevated. After one hour, hydrogen absorption began when the temperature reached about I90°C. Reaction was carried out at 200°C. Four hours thirty minutes after the initiation of temperature elevation, hydrogen absorption became late suddenly, and 7 hours after the initiation of temperature elevation, the reaction was stopped. After filtering off the catalyst, the weight of the reaction fluid was I45 g, and according to g.c. analysis, the content of diisopropylcarbinol was 87.2% and that of unreacted diisopropyl ketone was I0.3%.

## Comparative example 3

Operation was carried out in the same manner as in Example I except that a developed Raney nickel (a Ni-Si alloy made by Chisso Corporation) was used as catalyst in the form of hydrous slurry in a quantity of 5% by weight (7.9 g) based on the raw material, and temperature elevation was initiated. Thirty nine minutes after the initiation of temperature elevation, the temperature reached 130°C, at which the reaction was carried out. Seven hours after the initiation of temperature elevation, hydrogen absorption still continued slowly, but the reaction was stopped. After filtering off the catalyst, the weight of the reaction fluid was 148 g, and according to g.c. analysis, the content of diisopropylcarbinol was 66.8% and that of unreacted diisopropyl ketone was 30.9%.

## Comparative example 4

Operation was carried out in the same manner as in Comparative example 3 except that a developed Raney nickel (R-200, Ni-Aℓ alloy made by Nikko Rika Company) was used as catalyst, and temperature elevation was initiated. Thirty six minutes after the initiation of temperature elevation, the reaction temperature reached 130°C, at which the reaction was carried out. Seven hours after the initiation of temperature elevation, hydrogen absorption still continued slowly, but the reaction was stopped. After filtering off the catalyst, the weight of the reaction fluid was 150 g, and according to g.c. analysis, the content of diisopropylcarbinol was 50.3% and that of diisopropyl ketone was 46.8%.

## Comparative example 5

Operation was carried out in the same manner as in Example I except that a Cu-Cr-Mn catalyst (G-89, tradename of product made by Nissan Gardler Company) was used as catalyst in a quantity of 2% by weight (3.2 g) based on the raw material, and temperature elevation was initiated. Thirty nine minutes after the initiation of temperature elevation, the reaction temperature reached 130°C, at which the reaction was carried out. Seven hours after the initiation of reaction elevation, the reaction was completed. After filtering off the catalyst, the weight of the reaction fluid was 141.5 g, and according to g.c. analysis, the content of diisopropylcarbinol was 78.4% and that of unreacted diisopropyl ketone was 19.3%.

## Comparative example 6

The same Cu-Cr-Mn catalyst as used in Comparative example 5 was used as catalyst. The catalyst was fed into the autoclave in a quantity of 5% by weight (7.9 g) based on the raw material diisopropyl ketone 200 mℓ (158.8 g). Operation was carried out in the same manner as in Example I, and temperature elevation was initiated. Forty six minutes after the initiation of temperature elevation, the reaction temperature reached 150°C. At this temperature, the reaction was further continued. One hour and 40 minutes after the initiation of temperature elevation, hydrogen absorption ceased. The reaction was further carried out for 40 minutes and it was stopped. After cooling and pressure dropping, the autoclave was opened, and the catalyst was filtered off from the reaction fluid under pressure. The weight of the reaction fluid was 141.6 g, and according to g.c. analysis, the content of diisopropylcarbinol was 87.5% and that of unreacted diisopropyl ketone was 10.2%. Using the recovered catalyst as it was, all the same experiment was carried out. As a result, unlike the first time experiment, even when 7 hours lapsed, there was still hydrogen absorption in a slight quantity, but the reaction was stopped. The catalyst was filtered off from the reaction fluid. The weight of the reaction fluid was 160 g, and according to g.c. analysis, the content of diisopropylcarbinol was 88.2% and that of unreacted diisopropyl ketone was 9.7%. Using the recovered catalyst as it was, at the third experiment, all the same experiment was carried out. As a result, similarly even when 7 hours lapsed, there was still hydrogen absorption in a slight quantity, but the reaction was stopped. After filtering off the catalyst, the weight of the reaction fluid was 150 g, and according to g.c. analysis, the content of diisopropylcarbinol was 67.5% and that of unreacted diisopropyl ketone was 30.3%; thus the catalyst activity was considerably lost.

## Comparative example 7

Operation was carried out in the same manner as in Example I except that a nickel-diatomaceous earth (G-49B, tradename of product made by Nissan Gardler Company) was used as catalyst in a quantity of 2% by weight (3.2 g) based on the raw material, and temperature elevation was initiated. Thirty seven minutes after the initiation of temperature elevation, the reaction temperature reached 130°C. At this temperature the reaction was carried out. Until 3 hours and 4 minutes after the initiation of temperature elevation, there was hydrogen absorption although it was slow. One hour afrer there was no hydrogen absorption, the reaction was stopped. The catalyst was filtered off and the weight of the reaction fluid was 163 g. According to g.c. analysis the content of diisopropylcarbinol was 39.7% and that of unreacted diisopropyl ketone was 58.1%.

Comparative example 8

Operation was carried out in the same manner as in Example I except that as catalyst, cobalt-diatomaceous earth (G-67RS, tradename of a product made by Nissan Gardler Company) was used in a quantity of 5% by weight (7.9 g) based on the raw material, and temperature elevation was initiated. Thirty seven minutes after the initiation of temperature elevation, the reaction temperature reached 130°C. Even when one hour lapsed at this temperature, no hydrogen absorption occurred; thus the temperature was slowly raised, and hydrogen absorption occurred when the temperature reached 170°C. The reaction temperature was raised up to 180°C and 7 hours after the initiation of temperature elevation, there was still a slight hydrogen absorption, but the reaction was stopped. The catalyst was filtered off and the weight of the reaction fluid was 153 g. According to g.c. analysis, the content of diisopropylcarbinol was 82.0% and that of unreacted diisopropyl ketone was 15.9%.

Comparative example 9

Operation was carried out in the same manner as in Example I except that Cu-Cr-Ba catalyst (N-203 SB, tradename of product made by Nikki Kagaku Company) was used as catalyst in a quantity of 5% by weight (7.9 g) based on the raw material, and temperature elevation was initiated. Thirty seven minutes after the initiation of temperature elevation, the reaction temperature reached 140°C and hydrogen absorption began. Forty minutes after the initiation of temperature elevation, the reaction temperature reached 150°C at which the reaction was kept. Two hours and 40 minutes after the initiation of temperature elevation, hydrogen absorption suddenly became late, and 5 hours after the initiation of temperature elevation, the reaction was stopped. After cooling and pressure dropping, the autoclave was opened. The catalyst was filtered off under pressure from the reaction fluid. The weight of the reaction fluid was 146 g. According to g.c. analysis, the content of diisopropylcarbinol was 96.9% and that of unreacted diisopropyl ketone was 1.3%. Using the recovered catalyst as it was, all the same experiment was carried out. Unlike the first time experiment, even when 7 hours lapsed, there was still hydrogen absorption in a slight quantity, but the reaction was stopped. The catalyst was filtered off from the reaction fluid. The weight of the reaction fluid was 161 g. According to g.c. analysis, the weight of diisopropylcarbinol was 86.5% and that of unreacted diisopropyl ketone was 11.4%. Using the recovered catalyst as it was, at the third experiment, all the same experiment was carried out. Even when 7 hours lapsed, there was still a lazy hydrogen absorption, but the reaction was stopped. The catalyst was filtered off from the reaction fluid. The weight of the fluid was 161 g and according to g.c. analysis, the content of diisopropylcarbinol was 53.6% and that of unreacted diisopropyl ketone was 44.2%, that is, the catalyst activity was considerably lost.

## Claims

1. A process for producing diisopropylcarbinol by subjecting diisopropyl ketone to hydrogenation reaction, which process comprises using a catalyst containing ruthenium as a catalytically active substance.

2. A process according to claim I wherein said catalyst containing ruthenium as a catalytically active substance is a catalyst having ruthenium supported on a carrier selected from the group consisting of active carbon, active diatomaceous earth and alumina.

3. A process according to claim I wherein said hydrogenation reaction is carried out in a temperature range of 20˚ to 200˚C.

4. A process according to claim 2 wherein said hydrogenation reaction is carried out in a temperature range of 20˚ to 200˚C.

5. A process according to claim I wherein said hydrogenation reaction is carried out in a temperature range of 70˚ to I50˚C.

6. A process according to claim 2 wherein said hydrogenation reaction is carried out in a temperature range of 70˚ to I50˚C.

7. A process according to claim I wherein said hydrogenation reaction is carried out under a pressure of the atmospheric pressure to $9.8\times10^6$ Pa (I00 Kg/cm$^2$) gauge.

8. A process according to claim I wherein said hydrogenation reaction is carried out under a pressure of $9.8\times10^5$ to $4.9\times10^6$ Pa (10 to 50 Kg/cm$^2$) gauge.

9. A process according to claim I wherein said hydrogenation reaction is non-continuously carried out.

10. A process according to claim 9 wherein said catalyst containing ruthenium as a catalytically active substance is used in a quantity of 0.I to I0 parts by weight based on I00 parts by weight of diisopropyl ketone.

11. A process according to claim 9 wherein said catalyst containing ruthenium as a catalytically active substance is used in a quantity of 0.5 to 5 parts by weight based on I00 parts by weight of diisopropyl ketone.

12. A process according to claim 9 wherein said catalyst containing ruthenium as a catalytically active substance is used for a reaction time of 0.I to 9 hours.

13. A process according to claim 9 wherein said catalyst containing ruthenium as a catalytically active substance is used for a reaction time of 0.I to 7 hours.

14. A process according to claim I wherein said hydrogenation reaction is continuously carried out.

15. A process according to claim I4 wherein diisopropyl ketone is continuously fed to the reactor of said hydrogenation reaction in a quantity of 0.I to 5 parts by volume per hour based on I00 parts by volume of said catalyst.

**Revendications**

1. Procédé de préparation du diisopropylcarbinol selon lequel on soumet la diisopropyl cétone à une réaction d'hydrogénation, caractérisé en ce que l'on utilise un catalyseur contenant du ruthénium comme substance catalytiquement active.

2. Procédé selon la revendication 1, dans lequel ledit catalyseur contenant du ruthénium comme substance catalytiquement active est un catalyseur dans lequel le ruthénium est porté par un véhicule choisi dans le groupe consistant en charbon actif, terre active de diatomées et alumine.

3. Procédé selon la revendication 1, dans lequel ladite réaction d'hydrogénation est effectuée dans une plage de température de 20˚ à 200˚C.

4. Procédé selon la revendication 2, dans lequel ladite réaction d'hydrogénation est effectuée dans une plage de température de 20˚ à 200˚C.

5. Procédé selon la revendication 1, dans lequel ladite réaction d'hydrogénation est effectuée dans une plage de température de 70˚ à 150˚C.

6. Procédé selon la revendication 2, dans lequel ladite réaction d'hydrogénation est effectuée dans une plage de température du 70° à 150° C.

7. Procédé selon la revendication 1, dans lequel ladite réaction d'hydrogénation est effectuée à une pression allant de la pression atmosphérique à 9,8 x 10⁶ Pa (100 kg/cm²).

8. Procédé selon la revendication 1, dans lequel ladite réaction d'hydrogénation est effectuée à une pression de 9,8 x 10⁵ à 4,9 x 10⁶ Pa (10 à 50 kg/cm²).

9. Procédé selon la revendication 1, dans lequel ladite réaction d'hydrogénation est effectuée de manière non continue.

10. Procédé selon la revendication 9, dans lequel ledit catalyseur contenant du ruthénium comme substance catalytiquement active est utilisé en une quantité de 0,1 à 10 parties en poids pour 100 parties en poids de diisopropyl cétone.

11. Procédé selon la revendication 9, dans lequel ledit catalyseur contenant du ruthénium comme substance catalytiquement active est utilisé en une quantité de 0,5 à 5 parties en poids pour 100 parties en poids de diisopropyl cétone.

12. Procédé selon la revendication 9, dans lequel ledit catalyseur contenant du ruthénium comme substance catalytiquement active est utilisé pendant une durée de réaction de 0,1 à 9 heures.

13. Procédé selon la revendication 9, dans lequel ledit catalyseur contenant du ruthénium comme substance catalytiquement active est utilisé pendant une durée de réaction de 0,1 à 7 heures.

14. Procédé selon la revendication 1, dans lequel ladite réaction d'hydrogénation est effectuée de manière continue.

15. Procédé selon la revendication 14, dans lequel le réacteur de ladite réaction d'hydrogénation est alimenté de façon continue en diisopropyl cétone en une quantité de 0,1 à 5 parties en volume par heure pour 100 parties en volume dudit catalyseur.


## Ansprüche

1. Verfahren zur Herstellung von Diisopropylcarbinol durch Hydrierung von Diisopropylketon unter Verwendung eines Katalysators, der als katalytisch aktive Substanz Ruthenium enthält.

2. Verfahren nach Anspruch 1, worin der Katalysator, der Ruthenium als katalytisch aktive Substanz enthält, ein Katalysator ist, bei dem sich Ruthenium auf einem Träger befindet, der aus der aus Aktivkohle, aktiver Diatomeenerde und Aluminiumoxid bestehenden Gruppe ausgewählt ist.

3. Verfahren nach Anspruch 1, worin die Hydrierung innerhalb eines Temperaturbereichs von 20° bis 200° C durchgeführt wird.

4. Verfahren nach Anspruch 2, worin die Hydrierung innerhalb eines Temperaturbereichs von 20° bis 200° C durchgeführt wird.

5. Verfahren nach Anspruch 1, worin die Hydrierung innerhalb eines Temperaturbereichs von 70° bis 150° C durchgeführt wird.

6. Verfahren nach Anspruch 2, worin die Hydrierung innerhalb eines Temperaturbereichs von 70° bis 150° C durchgeführt wird.

7. Verfahren nach Anspruch 1, worin die Hydrierung unter einem Druck von Atmosphärendruck bis 9,8 x 10⁶ Pa (100 Kg/cm²) durchgeführt wird.

8. Verfahren nach Anspruch 1, worin die Hydrierung unter einem Druck von $9,8 \times 10^5$ bis $4,9 \times 10^6$ Pa (10 bis 50 Kg/cm²) durchgeführt wird.

9. Verfahren nach Anspruch 1, worin die Hydrierung nicht-kontinuierlich durchgeführt wird.

10. Verfahren nach Anspruch 9, worin der Ruthenium als katalytisch aktive Substanz enthaltende Katalysator in einer Menge von 0,1 bis 10 Gewichtsteilen, bezogen auf 100 Gewichtsteile Diisopropylketon, verwendet wird.

11. Verfahren nach Anspruch 9, worin der Ruthenium als katalytisch aktive Substanz enthaltende Katalysator in einer Menge von 0,5 bis 5 Gewichtsteilen, bezogen auf 100 Gewichtsteile Diisopropylketon, verwendet wird.

12. Verfahren nach Anspruch 9, worin der Ruthenium als katalytisch aktive Substanz enthaltende Katalysator während einer Reaktionsdauer von 0,1 bis 9 Stunden verwendet wird.

13. Verfahren nach Anspruch 9, worin der Ruthenium als katalytisch aktive Substanz enthaltende Katalysator während einer Reaktionsdauer von 0,1 bis 7 Stunden verwendet wird.

14. Verfahren nach Anspruch 1, worin die Hydrierung kontinuierlich durchgeführt wird.

15. Verfahren nach Anspruch 14, worin Diisopropylketon dem Hydrierungsreaktor kontinuierlich in einer Menge von 0,1 bis 5 Volumteilen pro Stunde, bezogen auf 100 Volumteile des Katalysators, zugeführt wird.